Europäisches Patentamt

European Patent Office

Office européen des brevets

(19)

(11) Veröffentlichungsnummer: **0 309 915 B1**

(12) # EUROPÄISCHE PATENTSCHRIFT

(45) Veröffentlichungstag der Patentschrift:
16.10.91 Patentblatt 91/42

(51) Int. Cl.$^5$: **C07C 33/046, C07C 29/88**

(21) Anmeldenummer: **88115571.7**

(22) Anmeldetag: **22.09.88**

(54) **Verfahren zur Entfernung von Formaldehyd aus wässrigen Lösungen von 2-Butindiol-1,4.**

(30) Priorität: **30.09.87 DE 3732955**

(43) Veröffentlichungstag der Anmeldung:
**05.04.89 Patentblatt 89/14**

(45) Bekanntmachung des Hinweises auf die
Patenterteilung:
**16.10.91 Patentblatt 91/42**

(84) Benannte Vertragsstaaten:
**BE DE FR GB IT NL**

(56) Entgegenhaltungen: ·
**DD-A- 214 605**

(56) Entgegenhaltungen:
**DE-A- 2 931 692**
**US-A- 3 129 252**
**US-A- 3 130 236**
**US-A- 3 232 996**

(73) Patentinhaber: **BASF Aktiengesellschaft**
**Carl-Bosch-Strasse 38**
**W-6700 Ludwigshafen (DE)**

(72) Erfinder: **Merger, Franz, Dr.**
**Max-Slevogt-Strasse 25**
**W-6710 Frankenthal (DE)**
Erfinder: **Horler, Hans, Dr.**
**Hainweg 20**
**W-6100 Darmstadt (DE)**

EP 0 309 915 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

# Beschreibung

Diese Erfindung betrifft ein neues Verfahren zur Abtrennung von Formaldehyd aus wäßrigen Lösungen von 2-Butindiol-1,4.

2-Butindiol-1,4 hat als wichtiges Zwischenprodukt, z.B. für die Herstellung von Butandiol-1,4, großtechnische Bedeutung erlangt. Man stellt 2-Butindiol-1,4 z.B. nach dem Reppe-Verfahren aus Acetylen und Formaldehyd her. Die dabei erhaltenen rohen wäßrigen Butindiollösungen werden für die Hydrierung zum Butandiol herangezogen. Da das bei der Ethinylierung entstehende Butindiol durch Acetalbildung die Umsetzung des Formaldehyds erschwert, weisen die rohen Butindiol-Lösungen einen Restgehalt an Formaldehyd von etwa 0,2 bis 2 Gew.% auf, der sich nachteilig auf die Hydrierung auswirkt. So beeinträchtigt der Formaldehyd den Hydrierkatalysator und durch die Bildung schwer abtrennbarer Nebenprodukte, wie 2-Methylbutandiol-1,4, die Ausbeute und Reinheit des Butandiols. Man hat deshalb schon verschiedene Verfahren zur Abtrennung von Formaldehyd aus wäßrigen Lösungen von Butindiol vorgeschlagen.

Bei dem in der US-PS 3130236 beschriebenen Verfahren verringert man den Formaldehyd-Gehalt, indem man die Butindiollösung mehrere Stunden unter Druck auf 125 bis 200°C erhitzt. Dieses Verfahren führt zu einer partiellen Verharzung und erfordert lange Reaktionszeiten sowie einen hohen Energieaufwand. Nimmt man die Entfernung des Formaldehyds nach den Angaben der US-PS 3232996 vor, indem man die Formaldehyd enthaltende Butindiollösung bei 80 bis 110°C mit alkalisch wirkenden Mitteln behandelt, so treten ebenfalls erhebliche Butindiolverluste durch Verharzung auf. In der DE-OS 2931692 wird ein Verfahren beschrieben, bei dem man die wäßrigen Lösungen von Butindiol mit Wasserstoffperoxid und alkalisch wirkenden Mitteln bei 50 bis 150°C behandelt. Die Entfernung kleiner Mengen Formaldehyd durch Umsetzung mit Wasserstoffperoxid erfolgt jedoch erst bei relativ hohem Alkaliüberschuß mit ausreichender Geschwindigkeit. Da vor der weiteren Verarbeitung der Butindiollösungen überschüssiges Alkali neutralisiert werden muß, wird auf diese Weise die Konzentration an Salzen so erhöht, daß sich bei nachfolgenden Destillationen Ausbeuteverluste an Butindiol bzw. Butendiol oder Butandiol nicht vermeiden lassen. Nach den Angaben der DD-PS 214605 wird Formaldehyd aus wäßrigen Butindiollösungen nach Behandlung mit Anionenaustauschern durch Umsetzung mit Ammoniak und Entfernung des gebildeten Hexamethylentramins mit Kationenaustauschern abgetrennt. Bei diesem Verfahren werden jedoch nicht tolerierbare Ionenaustauschermengen benötigt, und die Abtrennung gelingt nur in unbefriedigendem Maße.

Diese Verfahren erlauben es wegen ihrer Nachteile nicht, Formaldehyd aus wäßrigen Lösungen von 2-Butindiol-1,4 auf wirtschaftlich befriedigende Weise abzutrennen.

Es wurde nun gefunden, daß man Formaldehyd aus wäßrigen Lösungen von 2-Butindiol-1,4 auf technisch einfache und besonders wirksame Weise dadurch abtrennen kann, daß man zu der wäßrigen Butindiollösung Methanol und ein sauer wirkendes Mittel gibt und aus dem Gemisch bei höheren Temperaturen Dimethylformal abdestilliert.

Das neue Verfahren ist auf beliebige Formaldehyd enthaltende wäßrige Lösungen von 2-Butindiol-1,4 anwendbar. Besonders gut eignet es sich zur Behandlung der bei der großtechnischen Umsetzung von Acetylen mit wäßrigem Formaldehyd erhaltenen wäßrigen Lösungen, die einen Butindiolgehalt von 10 bis 80, vorzugsweise 30 bis 70, insbesondere 30 bis 50 Gew.% und einen Formaldehydgehalt von 0,1 bis 3, vorzugsweise 0,2 bis 2 Gew.% aufweisen.

Als sauer wirkende Mittel kommen anorganische oder organische Säuren, Kationenaustauscher oder sauer wirkende Zeolithe in Betracht. Als organische Säure sei z.B. Methansulfonsäure und als anorganische Säure z.B. Schwefelsäure genannt. Bevorzugt sind Kationenaustauscher, wie die im Handel unter der Bezeichnung Lewatit® SPC 118 oder Lewasorb® AC 10 erhältlichen stark sauren Kationenaustauscher (Hersteller : Bayer AG). Zweckmäßigerweise wendet man das sauer wirkende Mittel in einer Menge von 0,01 bis 3,0, vorzugsweise 0,04 bis 1,0 val/kg Butindiol an. Methanol wird in einer Menge von 0,05 bis 2,0, vorzugsweise 0,10 bis 0,80, insbesondere 0,15 bis 0,60 kg/kg Butindiol eingesetzt.

Man destilliert das sich in dem wäßrigen Gemisch bildende Dimethylformal bei höherer Temperatur ab. Dabei wählt man Temperaturen von 50 bis 180, vorzugsweise 90 bis 100°C und destilliert diskontinuierlich oder kontinuierlich bei Normaldruck. Man kann die Destillation aber auch im Unter- bzw. Überdruckbereich durchführen.

Nach dem Verfahren der Erfindung läßt sich Formaldehyd aus wäßrigen Lösungen von 2-Butindiol-1,4 besonders wirksam und auf technisch einfache Weise entfernen. Die hohe Selektivität und Effizienz des Verfahrens ist überraschend, da die Hydrolyse von Acetalen höherer Alkohole, wie Butindiol, bekanntlich schwieriger ist als die Hydrolyse von Dimethylformal. Es ist ferner überraschend, daß die Überführung von Formaldehyd aus Butindiolacetalen in Dimethylformal ohne schädigende Eingriffe am Butindiol verläuft, da dieses in saurem Medium bekanntlich zu Umlagerungsreaktionen neigt. Durch die erfindungsgemäße Behandlung sinkt die Ausbeute an Butindiol nicht ab, sie läßt sich vielmehr noch verbessern. Ein zusätzlicher Vorteil ist der, daß das üblicherweise in den wäßrigen Butindiollösungen enthaltene Natriumformiat bei der erfindungsgemäßen Behandlung in Methylformiat (bei gleichzeitiger Bildung des Natriumsalzes der zugegebenen Säure) umgewandelt und durch die Destillation ebenfalls entfernt wird. So wird nach dem erfindungsgemäßen Verfahren überraschenderweise eine Natriumformiatabreicherung auf bis zu 0,03% und eine Formaldehyd-Entfernung bis zu Restmengen von unter 100 ppm erreicht.

Die in den Beispielen genannten Prozente sind Gewichtsprozente.

Beispiel 1

Zu 500 g einer rohen wäßrigen Lösung von Butindiol mit der Zusammensetzung 47% Butindiol, 51,7% Wasser, 1,2% Formaldehyd und 0,17% Natriumformiat gibt man 0,27 kg Methanol/kg Butindiol und den im Handel unter der Bezeichnung Lewatit® SPC 118 erhältlichen Kationenaustauscher (0,17 val Säure/kg Butindiol). Das Gemisch wird dann unter Atmosphärendruck zum Sieden erhitzt. Bei einem Rücklaufverhältnis von 10 : 1 destilliert man über eine 50 cm Füllkörperkolonne pro Stunde 0,06 kg Destillat/kg Butindiol aus dem Reaktionsgemisch ab. Gleichzeitig wird dem Destillationssumpf die gleiche Menge an Methanol zugeführt. Nach 6 Stunden lassen sich in dem wäßrigen Gemisch noch 0,03% Formaldehyd und 0,03% Ameisensäure, bezogen auf die methanolfreie Lösung, nachweisen. Die wäßrige Butindiollösung kann nach Abtrennung des Ionenaustauschers direkt zur Hydrierung eingesetzt werden.

Beispiel 2

Man verfährt wie in Beispiel 1 angegeben, wobei man jedoch 0,54 kg Methanol/kg Butindiol zusetzt. Die Analyse der nach 6 Stunden erhaltenen wäßrigen Lösung ergibt einen Formaldehydgehalt von 0,005%, bezogen auf die methanolfreie Lösung.

Beispiel 3

Man verfährt wie in Beispiel 1 angegeben, wobei man jedoch pro Stunde 0,09 kg Destillat/kg Butindiol aus dem Reaktionsgemisch entfernt, während gleichzeitig die gleiche Menge an Methanol zugeführt wird. Nach 5 Stunden sind noch 0,02% Formaldehyd, bezogen auf die methanolfreie Lösung, nachweisbar.

Beispiel 4

Man verfährt wie in Beispiel 1 angegeben, wobei die Reaktion jedoch bei 80°C (Sumpftemperatur) im Unterdruck bei 600 mbar durchgeführt wird. Nach 3 Stunden werden noch 0,45% Formaldehyd, bezogen auf die methanolfreie Lösung, nachgewiesen.

Beispiel 5

Man verfährt wie in Beispiel 1 angegeben, wobei man jedoch als Säure den im Handel unter der Bezeichnung Lewasorb® AC 10 erhältlichen Kationenaustauscher (0,17 val Säure/kg Butindiol) einsetzt. Der nach 5 Stunden bestimmte Formaldehydgehalt liegt, bezogen auf die methanolfreie Lösung, bei 0,2% Formaldehyd.

Beispiel 6

Man verfährt wie in Beispiel 1 angegeben, wobei man jedoch als Säure Schwefelsäure (0,85 val/kg Butindiol) zusetzt. Nach 6 Stunden werden 0,06% Formaldehyd, bezogen auf die methanolfreie Lösung, bestimmt.

Beispiel 7

Man verfährt wie in Beispiel 1 angegeben, wobei man die Reaktion jedoch in Anwesenheit von Lewatit® SPC 118 (0,09 val Säure/kg Butindiol) durchführt. Der nach 6 Stunden bestimmte Restformaldehydgehalt beträgt 0,4%, bezogen auf die methanolfreie Lösung.

**Patentansprüche**

1. Verfahren zur Entfernung von Formaldehyd aus wäßrigen Lösungen von 2-Butindiol-1,4, dadurch gekennzeichnet, daß man zu der wäßrigen Butindiollösung Methanol und ein sauer wirkendes Mittel gibt und aus dem Gemisch bei Temperaturen von 50 bis 180°C Dimethylformal abdestilliert.

2. Verfahren nach Anspruch 1, dadurch gekennzeichnet, daß man zu der wäßrigen Butindiollösung 0,05 bis 2 kg Methanol und 0,01 bis 3 val Säure, jeweils berechnet auf 1 kg Butindiol, gibt.

3. Verfahren nach Anspruch 1, dadurch gekennzeichnet, daß man als sauer wirkendes Mittel anorganische oder organische Säuren, Kationenaustauscher oder sauer wirkende Zeolithe verwendet.

4. Verfahren nach Anspruch 1, dadurch gekennzeichnet, daß man eine wäßrige Lösung mit einem Butindiolgehalt von 10 bis 80 Gew.% und einem Formaldehydgehalt von 0,1 bis 3 Gew.% einsetzt.

**Claims**

1. A process for removing formaldehyde from aqueous solutions of 2-butyne-1,4-diol, which comprises adding to the aqueous butynediol solution methanol and an agent with an acidic action, and removing dimethylformal from the mixture by distillation at from 50 to 180°C.

2. A process as claimed in claim 1, wherein from 0.05 to 2 kg of methanol and from 0.01 to 3 eq of acid, in each case calculated on the basis of 1 kg of butynediol, are added to the aqueous butynediol solution.

3. A process as claimed in claim 1, wherein inorganic or organic acids, cation exchangers or zeolites with an acidic action are used as agent with an acidic action.

4. A process as claimed in claim 1, wherein an aqueous solution with a butynediol content of from 10 to 80% by weight and a formaldehyde content of from 0.1 to 3% by weight is employed.

## Revendications

1. Procédé pour l'élimination de formaldéhyde de solutions aqueuses de 2-butynediol-1,4, caractérisé en ce qu'on ajoute, à la solution aqueuse de butynediol, du méthanol et un agent acidifiant et en ce qu'on chasse le diméthylformal du mélange par distillation à une température de 50 à 180°C.

2. Procédé selon la revendication 1, caractérisé en ce qu'on ajoute, à la solution aqueuse de butynediol, de 0,05 à 2 kg de méthanol et de 0,01 à 3 val. d'acide, sur la base de 1 kg de butynediol.

3. Procédé selon la revendication 1, caractérisé en ce qu'on utilise, comme agent acidifiant, des acides minéraux ou organiques, des échangeurs de cations ou des zéolithes à action acide.

4. Procédé selon la revendication 1, caractérisé en ce qu'on traite une solution aqueuse ayant une teneur en butynediol de 10 à 80% en poids et une teneur en formaldéhyde de 0,1 à 3% en poids.